# EUROPEAN PATENT SPECIFICATION

(11) **EP 3 967 359 B1**
(45) Date of publication and mention of the grant of the patent: **05.02.2025**
(21) Application number: 20801865.5
(22) Date of filing: 02.06.2020
(51) Int. Cl.: A61M 37/00, A61N 1/30, A61N 1/04, A61N 1/32

(54) **IONTOPHORESIS ADMINISTRATION DEVICE**
IONTOPHORESEVERABREICHUNGSVORRICHTUNG
DISPOSITIF D'ADMINISTRATION PAR IONTOPHORÈSE

(43) Date of publication of application: 16.03.2022
(73) Proprietor: Shanghai Futai Technology Co., Ltd., Shanghai 201821 (CN)
(72) Inventor: YANG, Feng, Shanghai 200433 (CN)
(74) Representative: Lavoix
(86) International application number: PCT/CN2020/093888
(87) International publication number: WO 2020/224664

(56) References cited:
- EP-A1- 3 378 527
- EP-B1- 0 681 497
- CN-A- 101 282 758
- US-A- 5 464 387
- US-A1- 2009 186 072
- US-A1- 2010 106 075
- US-A1- 2011 082 411

## Description

### TECHNICAL FIELD

The application relates to an administration device, in particular to an iontophoresis administration device.

### BACKGROUND

Percutaneous administration refers to a method of administration on a skin surface to allow a medicament to pass through the skin at a certain rate and enter systemic circulation to produce systemic or local therapeutic effect. Because the percutaneous administration method has the advantages of not being affected by factors such as food in the digestive tract and continuously controlling the administration rate, it has broad application prospects.

In the process of percutaneous administration, the skin stratum corneum has a barrier function, which makes it difficult for the current penetration rate and penetration amount through the skin surface to achieve expected effect. A traditional scheme for improving the penetration rate and penetration amount is, for example, a microneedle percutaneous administration method, that is, a microneedle array is set on an administration carrier to make the microneedles penetrate the skin stratum corneum, thereby improving the penetration rate of the percutaneous administration method. The above-mentioned microneedle percutaneous scheme has relatively high requirements for the length of the microneedles, and if they are not well controlled, it is easy to damage the skin and cause pain.

Another traditional scheme for improving the percutaneous administration is, for example, an iontophoresis administration method, that is, a non-invasive method that uses charged charges to push active medicaments with polarity into the skin by electromotive force. In the traditional iontophoresis administration method, electrically driving single-point electrode or multiple administration carriers with electrodes are usually set for administration. The above iontophoresis administration method either has no obvious penetration effect or is easy to cause the skin burns.

Documents EP 0 681 497 B1, EP 3 378 527 A1, US 2011/082411 A1 and US 2010/106075A1 disclose iontophoresis administration devices.

Therefore, the traditional percutaneous administration schemes have some shortcomings: either the penetration effect is not obvious, or they are easy to cause the skin burns or pain and other skin injuries.

### SUMMARY OF THE INVENTION

According to exemplary embodiments of the present disclosure, there is provided an iontophoresis administration device that not only can improve the penetration efficiency, but also does not cause skin injury easily.

In the present disclosure, there is provided an iontophoresis administration device as presented in the appended set of claims.

It should be understood that the contents described in the summary section are not intended to limit key or important features of the embodiments of the present disclosure nor intended to limit the scope of the present disclosure. Other features of the present disclosure will be readily understood by the following description.

### BRIEF DESCRIPTION OF THE FIGURES

The above and other features advantages and aspects of various embodiments of the present disclosure will become more apparent when taken in conjunction with the accompanying figures and with reference to the following detailed description. In the figures, identical or like reference numerals indicate identical or like elements, wherein:
Fig. 1 shows a schematic diagram of an iontophoresis administration device 100 according to some embodiments of the present disclosure;
Fig. 2 shows a partial structural schematic diagram of an iontophoresis administration device 200 according to some embodiments of the present disclosure;
Fig. 3 shows a schematic diagram of an iontophoresis administration device 300 according to some embodiments of the present disclosure;
Fig. 4 shows a schematic diagram of an iontophoresis administration device 400 according to some embodiments of the present disclosure;
Fig. 5 shows a schematic diagram of an iontophoresis administration device 500 according to some embodiments of the present disclosure;
FIG. 6 shows a schematic diagram of an iontophoresis administration device 600 according to some embodiments of the present disclosure; and
Fig. 7 shows a schematic diagram of an iontophoresis administration device 700 according to some embodiments of the present disclosure;

In the various figures, the same or corresponding reference numerals indicate the same or corresponding parts.

### DETAILED DESCRIPTION

Embodiments of the present disclosure will be described in more detail below with reference to the accompanying figures. While certain embodiments of the present disclosure are shown in the accompanying figures, it should be understood that the present disclosure may be embodied in various forms and should not be construed as being limited to the embodiments set forth herein, on the contrary these embodiments are provided for a more thorough and complete understanding of the present disclosure. It should be understood that the figures and embodiments of the present disclosure are for exemplary purposes only and are not intended to limit the scope of protection of the present disclosure.

In the description of embodiments of the present disclosure, the term "comprising" and similar terms should be understood as open comprising, i.e., "comprising but not limited to". The term "based" should be understood as "based at least in part". The term "an embodiment" or "the embodiment" should be understood as "at least one embodiment". The terms "first", "second", etc. may refer to different or identical objects. Other definitions, both explicit and implicit, may be included below.

As mentioned above traditional iontophoresis administration scheme is for example provided with a single electrode or multiple administration carriers each with electrode, the electrode is electrically connected with a power supply. Before administration, measuring skin resistance of the organism to be administered, and setting a safe power supply voltage and current according to the measured resistance, or setting a safe power supply voltage and current according to a general empirical value of the skin resistance, so as to ensure that the skin of the organism to be administered is not injured.

In the above-mentioned traditional iontophoresis administration scheme, on the one hand, with the advancement of the administration process, skin state (such as water content and medicament amount accumulated in the skin) of the administrated area will change. At the same time, the skin resistance of the administrated area also changes as the skin state changes. Therefore, the power supply voltage and current value set before administration do not take into account the change amount of the skin resistance during administration, therefore, it will not be compatible with the changed skin resistance during the administration. For example, during the administration process, the water content or the medicament amount in the skin increases, and the skin resistance decreases. When the preset power supply voltage remains unchanged, the current intensity flowing through the skin will increase, which is likely to cause irritation and burns to the skin.

To address at least one of the above problems and one or more of other potential problems, embodiments of the present disclosure propose an iontophoresis administration device. The device comprises: a power supply for generating electricity required to penetrate a medicament to be penetrated into an administered area of an organism; an integrated dielectric layer for covering the administered area, wherein the integrated dielectric layer comprises: a gel, and the polar, free-state medicament to be penetrated; and a plurality of electrodes for electrically connecting with the power supply and the integrated dielectric layer respectively, such that the electricity generated by the power supply flows through the administered area and at least part of the integrated dielectric layer respectively, wherein the at least part of the integrated dielectric layer has a predetermined resistance value.

In the iontophoresis administration device provided by the present disclosure, since the electricity generated by the power supply is caused to flow through the administered area and the at least part of the integrated dielectric layer having the predetermined resistance value respectively, that is, by connecting the integrated dielectric layer having the predetermined resistance value in parallel with the skin resistance of the administered area, the current flowing through the administered area is shunted, thereby avoiding excessive current intensity irritation or burns to the skin. In addition, according to the principle of the parallel circuit, the ratio of the current shunted by the dielectric layer is related to the ratio between the predetermined resistance value of the dielectric layer and the skin resistance of the administration area. Therefore, when the resistance value of the dielectric layer is constant, the current shunted by the dielectric layer also changes as the skin resistance of the administration area changes. Since the skin state changes as different individuals are different, therefore the iontophoresis administration device of the present disclosure can not only solve the technical problems of irritation or burns to the skin caused by the increase of the current intensity caused by the decrease of the skin resistance in the administration process, moreover can adaptively adjust the current flowing through the skin according to the different skin conditions of different individuals, in a simple and convenient way. Furthermore, in the iontophoresis administration device, since a part of the current flows through the integrated dielectric layer which is between the electrodes, contains the gel with the free-state medicament, and has the predetermined resistance value, the integrated dielectric layer and its gel covering the surface of the administered area will generate heat. The epidermis comprises a stratum corneum, a granular layer, a spinous layer and a basal layer from outside to inside. The key to percutaneous absorption in the epidermis lies in "skin barrier". The skin barrier comprises a stratum corneum and a sebum film. Under normal circumstances, appropriate increase in skin temperature is beneficial to improving the efficiency of the medicament to be penetrated penetrating the "skin barrier". Therefore, the heat generated by the current flowing through the integrated dielectric layer is beneficial to improve the efficiency of the medicament to be penetrated through the "skin barrier".

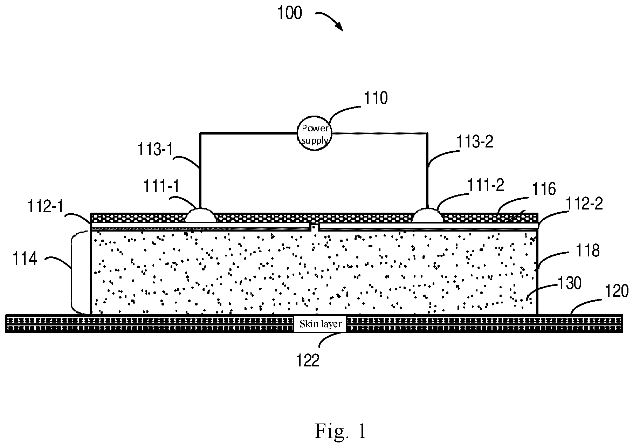

Fig. 1 shows a schematic diagram of an iontophoresis administration device 100 according to some embodiments of the present disclosure. In the iontophoresis administration device 100 of this embodiment, comprises a power supply 110, a plurality of electrodes 112-1 and 112-2, and an integrated dielectric layer 114. The integrated dielectric layer 114 further comprises a gel 118 and a polar, free-state medicament to be penetrated 130. The integrated dielectric layer 114 overlays the administered area 122 of the organism 120 and adapts to the profile of the administered area 122. In some embodiments, the administered area 122 is for example a local skin of a human body such as but not limited to facial skin. The integrated dielectric layer 114 is provided, for example, in the profile of a mask. In some embodiments, the integrated dielectric layer 114 further comprises a mesh structure (not shown). In some embodiments, the polar, free-state medicament to be penetrated 130 is dispersed in the cross-linked network structure of the gel 118. In some embodiments, the polar, free-state medicament to be penetrated 130 is distributed on the surface of the gel 118. In some embodiments, the gel 118 comprises collagen or the gel 118 is a collagen gel.

With respect to the power supply 110, it is used to generate electricity required to penetrate the medicament to be penetrated into the administered area. In some embodiments, the iontophoresis administration device 100 comprises one power supply. In some embodiments, the iontophoresis administration device 100 comprises a plurality of power supplies. The plurality of power supplies may be connected in series and/or in parallel with each other for providing appropriate voltage and current to penetrate the medicament to be penetrated into the administered area. The electricity generated by the power supply 100 may be constant or variable. In some embodiments, the settings of the power supply time, the power supply voltage, and the current intensity of the power supply are associated with the dosage of the medicament to be penetrated. In some embodiments, the power supply voltage amplitude, the current intensity, the power supply time, the frequency, the duty cycle, etc. of the power supply are adjusted according to the size of the dosage, different administration modes (e.g., instantaneous dosage administration mode, constant administration mode, pulse administration mode, etc.).

In some embodiments, the electricity provided by the power supply 110 is an alternating current. This is because, on the one hand, direct current is easy to cause local anesthetic effect on the skin, which is easy to cause imperceptible skin injury to the medicament recipient, and on the other hand, direct current is easy to cause the accumulation of electric charge in the skin layer, which in turn leads to a decrease in the rate and total amount of the medicament to be penetrated 130 delivered over time. By alternating current direction of the alternating current provided by the power supply 110, the accumulation of capacitive charge in the skin layer can be solved, thereby facilitating the improvement of the penetration efficiency and the total amount of the medicament. In some embodiments, the current intensity of the alternating current is less than or equal to 5 mA. In some embodiments, the current intensity of the alternating current is greater than or equal to 0.01 mA and less than 5 mA, and the current intensity is set in a range that not only avoids skin injury, but also ensures sufficient electrical potential energy required to penetrate the medicament to be penetrated into the administered area.

With regard to the electrodes 112, they are configured as ion penetration electrodes. The plurality of electrodes 112 are used to connect two output ends of the power supply 110 to the integrated dielectric layer 114 respectively, so that the electricity generated by the power supply flows through the administered area and at least part of the integrated dielectric layer respectively. For example, as shown in Fig. 1, the electrodes 112 comprise a first electrode 112-1 and a second electrode 112-2, wherein the first electrode 112-1 is electrically connected to a first end of the power supply 110 via a connector 111-1 and a wire 113-1, and the second electrode 112-2 is electrically connected to a second end of the power supply 110 via a connector 111-2 and a wire 113-2. The electricity generated by the power supply 110 flows through the administered area 122 and at least part of the integrated dielectric layer (e.g., the part of the integrated dielectric layer between the electrical contact of the first electrode 112-1 and the integrated dielectric layer 114 to the electrical contact of the second electrode 112-2 and the integrated dielectric layer) respectively. The part of the integrated dielectric layer 114 through which the current flows has the predetermined resistance value.

As far as the administered subjects are concerned, due to differences of individual skin (for example, differences in the ratio of water to oil, dryness, pores, etc.), there are certain differences in individual skin resistance values. Because the current flowing through the skin may burn the skin due to the heat generated by the skin resistance value, in order to avoid causing injury to the skin of the administered object, the traditional iontophoresis administration scheme usually strictly limits the output current intensity based on the skin resistance value under extreme condition, which will lead to the side effect of reducing the percutaneous administration efficiency. In the above scheme of the present disclosure, by covering the integrated dielectric layer 130 which is integral, has the predetermined resistance value and contains the polar, free-state medicament to be penetrated under the plurality of independent electrodes 112, shunting the current flowing through the skin of the administered area 122 by utilizing the integrated dielectric layer in parallel with the skin resistance of the administered area 122, thereby reducing the current intensity flowing through the skin and effectively avoiding skin irritation or burns under a given power supply voltage.

With respect to the predetermined resistance value of the integrated dielectric layer 114, it is assumed that the resistance of the integrated dielectric layer through which current flows is R1 and the skin resistance of the administered area 122 is R2. According to the principle of resistance parallel connection, if R1 > R2, most of the current provided by the power supply is used to activate the active substances in the skin. If R1 < R2, most of the current provided by the power supply is used to push the active substance dispersed in the gel into the skin. In the invention R1is set to 10%-90% of R2. In some embodiments, R1 is set to be greater than 100 ohms and less than or equal to 100K ohms, and in some embodiments, R1 is set to be greater than 1K ohms and less than or equal to 12K ohms, e.g., 10K ohms.

With respect to the electrodes 112, they may be sheet-shaped electrodes, dot-shaped electrodes, layered electrodes or another shapes. In some embodiments, the plurality of electrodes 112 are a plurality of flexible conductive electrode films separated by a predetermined interval. The conductive electrode film is, for example, a low-resistance conductive film, a conductive carbon film, and a carbon-based conductive film. The flexible conductive electrode film is prepared by mixing polyethylene with polymer superconducting nano carbon black, curing medicament, auxiliary medicament and the like. The ion penetration electrodes are arranged in the form of flexible conductive electrode films, which have the advantages of uniform and stable resistance value, good bending performance and flexibility performance, and are beneficial to make the integrated dielectric layer 114 and the electrodes better fit the different external contour of the administered area, and are not easy to be electric corrosion. Therefore, compared with the traditional sheet-shaped ion penetration electrodes, they have obvious advantages. In some embodiments, the plurality of electrodes in the form of flexible conductive electrode films may be attached to a back substrate layer 116 by an adhesive, and the electrodes in the form of flexible conductive electrode films are separated from each other on the back substrate layer 116 by the predetermined interval. The predetermined interval is, for example, 0.1 to 10mm, preferably 1 to 2mm. With this arrangement, the penetration efficiency can be improved by controlling the coverage area of the ion penetration electrodes.

With respect to the gel 118, it comprises a matrix, an active medicament and an additive. The gel 118 is formed by cross-linking, medicament dispersion, curing and the like. Wherein, the active medicament is the polar and free-state medicament to be penetrated 130. The additive comprises, for example, but not limited to, penetration enhancer, pharmaceutical solvent, etc. In some embodiments, the gel 118 comprises, for example, at least one or more of the following components: polyethylene glycol, polyvinyl alcohol, polyhydroxyethyl methacrylate, polyacrylic acid, polymethacrylic acid, gelatin, alginic acid. Experiments show that the gel composed of the above components can not only form a polymer cross-linking network structure with certain strength, but also make the medicament to be penetrated dispersed freely in the gel. This arrangement facilitates not only shunting the current flowing through the skin of the administered area 122, but also significantly improving the controlled release effect of the medicament to be penetrated 130. The experimental results show that after penetration administration of the gel provided by the present disclosure, administration concentration of the medicament 130 can be stable, and the bio-availability is ideal.

With respect to the medicament to be penetrated 130, it is for example but not limited to the medicament for pain relief, treatment of arthritis or asthma, hormone regulation, cosmetic and the like purposes. In some embodiments, the medicament to be penetrated 130 has a molecular weight less than or equal to 10,000 daltons. This is because studies have shown that the medicament to be penetrated 130 with molecular weight less than or equal to 10,000 daltons can easily enter the skin through intercellular space, which is beneficial to improve the penetration efficiency. For example, the polar, free-state medicament 130 with molecular weight less than or equal to 10,000 daltons is uniformly dispersed in the gel 118, released from the gel 118 under the action of the charge provided by the electrode 112, diffused to the skin stratum corneum, and then extended to the dermis through the epidermis and even into the capillaries of the skin.

In some embodiments, the medicament to be penetrated 130 comprises vitamin C and arbutin. The arbutin in the medicament to be penetrated 130 is beneficial to inhibit tyrosinase in the skin, block melanin formation, accelerate melanin decomposition and excretion, and has antiinflammatory effect at the same time. The vitamin C in the medicament to be penetrated 130 has antioxidant effect, which is beneficial to inhibit the formation of pigment spots and promote the regression of pigment spots. Experiments show that, the medicament to be penetrated 130 comprises both vitamin C and arbutin components, and is polarized and uniformly dispersed in a free-state in the gel 118 of the integrated dielectric layer 114, an electric current is applied via an electrode of the iontophoresis administration device 100 so that the polar, free-state medicament to be penetrated 130 is introduced into the administrated area 122, and the administrated area 122 and the surrounding area can be rapidly whitened, and the whitening effect can last for a considerable period of time. In some embodiments, the medicament to be penetrated 130 containing vitamin C and arbutin is uniformly distributed in the gel 118 of an integrated dielectric layer 114 disposed in the shape of a facial mask for whitening the human face by iontophoresis.

In some embodiments, the medicament to be penetrated 130 comprises vitamin C and tranexamic acid. The tranexamic acid in the medicament to be penetrated 130 is used to inhibit tyrosinase activity, thereby inhibiting melanin formation. Experiments show that, the medicament to be penetrated 130 comprises both vitamin C and tranexamic acid components, and is polarized and uniformly dispersed in a free-state in the gel 118 of the integrated dielectric layer 114, and an electric current is applied through the electrode of the iontophoresis administration device 100, so that the polar, free-state medicament 130 to be penetrated is introduced into the administrated area 122, and dark spots in the administrated area 122 can be effectively removed. In some embodiments, the medicament to be penetrated 130 containing vitamin C and tranexamic acid is uniformly distributed in the gel 118 of the integrated dielectric layer 114 disposed in the shape of a facial mask for freckle removal of a human face by iontophoresis.

The iontophoresis administration device 100 further comprises a back substrate layer 116 for overlaying the plurality of electrodes 112, the back substrate layer 116 is an insulation material. The plurality of connectors 111-1 and 111-2 are used for electrically connecting the corresponding electrodes to the power supply respectively, each of the plurality of connectors 111-1 and 111-2 is at least partially disposed in the back substrate layer 116.

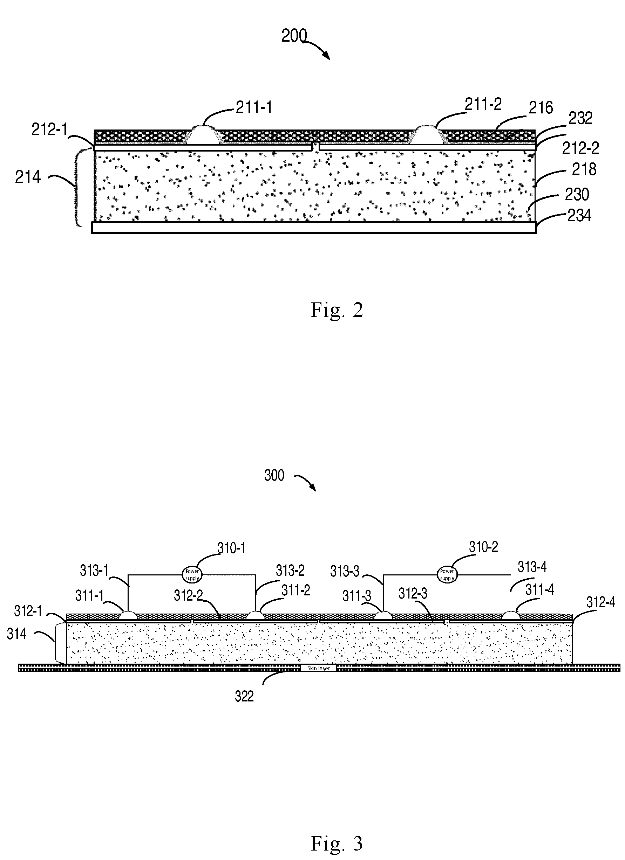

Fig. 2 shows a partial structural schematic diagram of an iontophoresis administration device 200 according to some embodiments of the present disclosure. In the iontophoresis administration device 200 of this embodiment, comprises a laminated structure. The back substrate layer 216 is located on the surface layer of the laminated structure. Below the back substrate layer 216 is an adhesive layer 232 for bonding the back substrate layer 216 and the flexible conductive electrode films 212-1 and 212-2. Below the adhesive layer 232 are flexible conductive electrode films 212-1 and 212-2. Below the flexible conductive electrode films 212-1 and 212-2 is a gel layer 218 containing the medicament to be penetrated 230. Below the gel layer 218 is a stripping layer 234 for protecting the active component in the gel layer 218 from contamination. When using the iontophoresis administration device 200, the stripping layer 234 will be peeled so that the gel layer 218 is overlaying over the skin of the administered area. The adhesive layer 232 comprises a conductive adhesive, such as but not limited to, pressure-sensitive adhesive, oil-based adhesive, water-based adhesive, hot-melt adhesive, etc. containing conductive fillers such as carbon or metal.

The plurality of connectors 211-1 and 211-2 made of a conductive material are provided on the back substrate layer 216, each connector being at least partially provided in the back substrate layer 216 for coupling the electricity from the power supply to corresponding flexible conductive electrode films 212-1 and 212-2. Connection modes between the connector and the power supply or the wire connected to the power supply may be in various ways, such as but not limited to mechanical coupling connection such as snap connection, magnet action connection, bonding or welding. The connection modes of the snap connection and the magnet action connection are convenient for adjusting the connection mode between power supply and electrodes. For example, each connector 211 is a pair of connector components (not specifically shown) that can be snapcoupled, one connector component is connected to the wire connected to the power supply, and the other connector component is connected to a flexible conductive electrode film. Through the snap coupling of the two connector components, the electrical connection between the wire connected to the power supply and the flexible conductive electrode film is realized. For another example, each connector 211 is a pair of magnet connector components (not specifically shown) capable of attracting each other, one magnet connector component is connected to the wire connected to the power supply, and the other magnet connector component is connected to a flexible conductive electrode film. The two magnet connector components are jointed together by magnet action, thereby realizing electrical connection between the wire connected to the power supply and the flexible conductive electrode film.

Fig. 3 shows a schematic diagram of an iontophoresis administration device 300 according to some embodiments of the present disclosure. In the iontophoresis administration device 300 of this embodiment, comprises a first power supply 310-1, a second power supply 310-2, a plurality of electrodes 312 (e.g., a first electrode 312-1, a second electrode 312-2, a third electrode 312-3, a fourth electrode 312-4), and an integrated dielectric layer 314. The integrated dielectric layer 314 comprises a gel and a polar, free-state medicament to be penetrated. The integrated dielectric layer 314 overlays the administered area 322 of the organism. For example, the first electrode 312-1, the second electrode 312-2, the third electrode 312-3 and the fourth electrodes 312-4 are respectively covered on different sub-areas in the administered area 322.

As shown in Fig. 3, the first electrode 312-1 is electrically connected to a first end of the first power supply 310-1 via s first connector 311-1 and s first wire 313-1, and the second electrode 312-2 is electrically connected to a second end of the first power supply 310-1 via a second connector 311-2 and a second wire 313-2. In addition, the third electrode 312-3 is electrically connected to a first end of the second power supply 310-2 via a third connector 311-3 and a third wire 313-3, and the fourth electrode 312-2 is electrically connected to a second end of the second power supply 310-2 via a fourth connector 311-4 and a fourth wire 313-4. In the above scheme, the first power supply and the second power supply are provided, the first electrode and the second electrode are electrically connected to the first power supply, and the third electrode and the fourth electrode are electrically connected to the second power supply. The iontophoresis administration of different sub-areas of the administered area 322 may be controlled independently of each other. For example, when the first power supply 310-1 supplies power, the second power supply 310-2 stops supplying power. For another example, the power supply voltage amplitude, current intensity, frequency, duty cycle and/or power supply time of the first power supply 310-1 are different from the power supply voltage amplitude, current intensity, frequency, duty cycle and/or power supply time of the second power supply 310-2, so that the medicament can be administered separately according to the different characteristics of the different sub-areas of the administered area 322. For example, individuals with mixed skin have oily skin in the T-shaped area and dry skin in the U-shaped area. For another example, there is usually a difference in sensitivity between the skin in the eye area and the skin in the cheek area. By arranging independent power supplies in the same iontophoresis administration device 300 to respectively supply power to the electrodes at different positions corresponding to different skin sub-areas, it is possible to set matched power supply parameters for percutaneous administration for different skin types in different skin sub-areas.

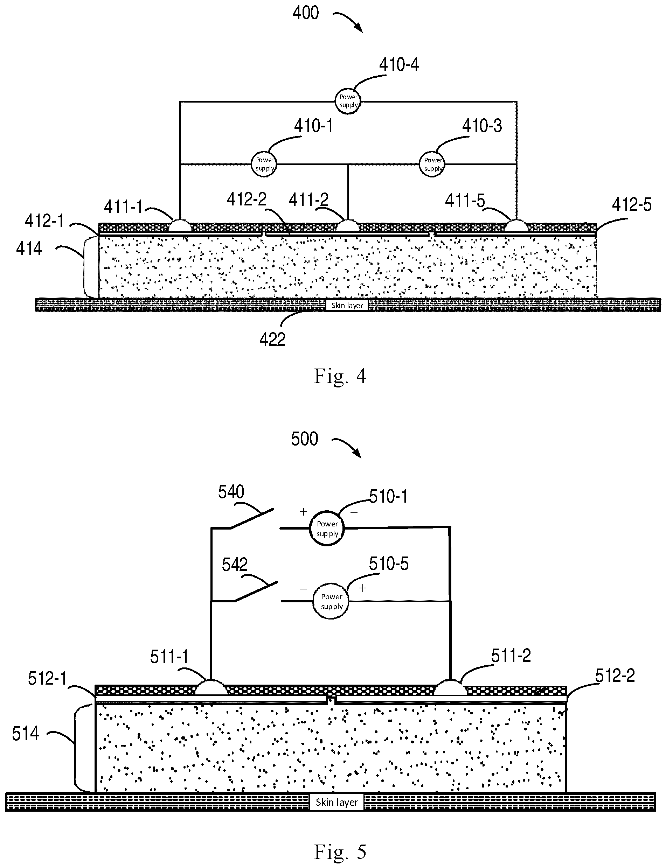

Fig. 4 shows a schematic diagram of an iontophoresis administration device 400 according to some embodiments of the present disclosure. In the iontophoresis administration device 400 of this embodiment, comprises a first power supply 410-1, a third power supply 410-3, a fourth power supply 410-4, a plurality of electrodes 412 (e.g., a first electrode 412-1, a second electrode 412-2, a fifth electrode 412-5), and an integrated dielectric layer 414. The integrated dielectric layer 414 comprises a gel and a polar, free-state medicament to be penetrated. The integrated dielectric layer 414 overlays the administered area 422 of the organism.

As shown in Fig. 4, the first electrode 412-1 is electrically connected to a first end of the first power supply 410-1 via a first connector 411-1 and a wire, and the second electrode 412-2 is electrically connected to a second end of the first power supply 410-1 via a second connector 411-2 and a wire. In addition, a first end of the third power supply 410-3 is electrically connected to the second end of the first power supply 410, and a second end of the third power supply 410-3 is electrically connected the fifth electrode 412-5 via a fifth connector 411-5. In addition, a first end of the fourth power supply 410-4 is electrically connected to the first end of the first power supply 410-1, and a second end of the fourth power supply 410-4 is electrically connected to the second end of the third power supply 410-3. In the above scheme, the first power supply 410-1 and the third power supply 410-3 are connected in series and then connected in parallel with the fourth power supply 410-4 for supplying power to the first electrode 412-1, the second electrode 412-2 and the fifth electrode 412-5, so that the electricity flows through the part of the integrated dielectric layer 414 and the skin of the administered area 422 respectively. The polar, free-state medicament to be penetrated, dispersed in the skeleton structure of the gel, is introduced into the skin of the administered area 422 under the action of an electric charge.

In the above scheme, by connecting two or more power supplies in series, the voltage amplitude required for iontophoresis administration can be adjusted. By connecting two or more power supplies in parallel, the current intensity required for iontophoresis administration can be adjusted. The first electrode 412-1, the second electrode 412-2, and the fifth electrode 412-5 are respectively connected to different output ends of the power supplies connected in series and parallel to each other, so that the electric parameters of iontophoresis administration between the first electrode 412-1 and the second electrode 412-2 and the electric parameters of iontophoresis administration between the second electrode 412-2 and the fifth electrode 412-5 are relatively independent and controllable, and have certain correlation. So as to realize personalized control of percutaneous administration.

Fig. 5 shows a schematic diagram of an iontophoresis administration device 500 according to some embodiments of the present disclosure. In the iontophoresis administration device 500 of this embodiment, comprises at least a first power supply 510-1, a fifth power supply 510-5, a first switch 540, a second switch 542, a plurality of electrodes 512 (e.g., a first electrode 512-1, a second electrode 512-2), an integrated dielectric layer 514, and a controller (not shown). The integrated dielectric layer 514 has the same structure as the integrated dielectric layers shown in Figs. 1-4 and will not be described here. The first power supply 510-1 and the fifth power supply 510-5 are direct current power supplies. In some embodiments, the current intensity of the direct current is less than or equal to 5 mA. In some embodiments, the current intensity of the direct current is greater than or equal to 0.01 mA. For example, the current intensity is greater than or equal to 0.1 mA and less than the safe current threshold of the skin. The first switch 540 and the second switch 542 are for example but not limited to relays that are turned on or off by the controller (not shown).

As shown in Fig. 5, the first electrode 512-1 is electrically connected to a positive electrode of the first power supply 510-1 at least via a first connector 511-1 and the first switch 540, and the second electrode 512-2 is electrically connected to a negative electrode of the first power supply 510-1 at least via a second connector 511-2. The first electrode 512-1 is also electrically connected to a negative electrode of the fifth power supply 510-5 via the second switch 542, and the second electrode 512-2 is also electrically connected to a positive electrode of the fifth power supply 510-5. That is, after the first power supply 510-1 and the first switch 540 are connected in series, they are connected in antiphase and parallel with the series circuit of the fifth power supply 510-5 and the second switch 540, and then electrically connected to the first electrode 512-1 and the second electrode 512-1 respectively.

For example, in a first time period, the controller outputs a high-level first signal to turn on the first switch 540, and simultaneously outputs a low-level second signal to turn off the second switch 542, so that the positive electrode of the first power supply 510-1 is electrically connected with the first electrode 512-1, and the negative electrode of the first power supply 510-1 is electrically connected with the second electrode 512-2, that is, the medicament to be penetrated is introduced with the current in a first direction in the first time period. And in the second time period, the controller outputs a low-level first signal to turn off the first switch 540, and simultaneously outputs a high-level second signal to turn on the second switch 542, so that the negative electrode of the fifth power supply 510-5 is electrically connected with the first electrode 512-1 and the positive electrode of the fifth power supply 510-5 is electrically connected with the second electrode 512-2 in the second time period, that is, the medicament to be penetrated is introduced with the current in a second direction in the second time period. By adopting the above solution, multiple direct current power supplies combining with switches are used to solve the problem of the accumulation of capacitive charges in the skin layer, thereby improving the penetration efficiency and total amount of the medicament.

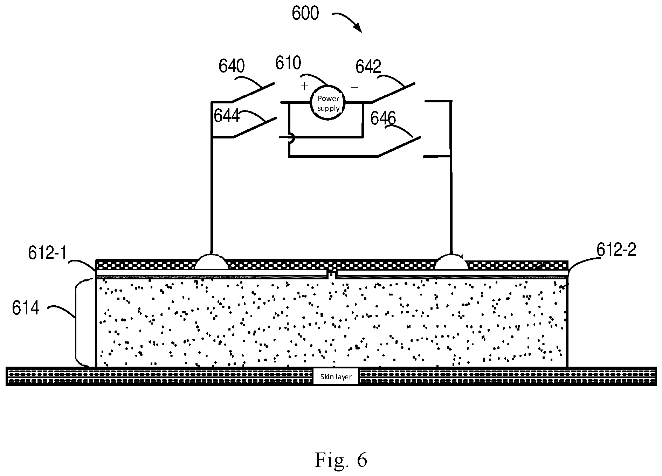

Fig. 6 shows a schematic diagram of an iontophoresis administration device 600 according to some embodiments of the present disclosure. In the iontophoresis administration device 600 of this embodiment, comprises at least a first power supply 610-1, a first switch 640, a second switch 642, a third switch 644, a fourth switch 644, a plurality of electrodes 612 (e.g., a first electrode 612-1, a second electrode 612-2), an integrated dielectric layer 614, and a controller (not shown). The integrated dielectric layer 614 has the same structure as the integrated dielectric layers shown in Figs. 1-5 and will not be described here. The first power supply 610-1 is a direct current power supply. The first switch 640, the second switch 642, the third switch 644 and the fourth switch 644 are for example but not limited to relays that are turned on or off by the controller (not shown).

As shown in Fig. 6, the first electrode 612-1 is electrically connected to a positive electrode of the first power supply 610-1 at least via the first switch 640, and the first electrode 612-1 is electrically connected to a negative electrode of the first power supply 610-1 at least via the third switch 644. The second electrode 612-2 is electrically connected to the negative electrode of the first power supply 610-1 via at least the second switch 642, and the second electrode 612-2 is electrically connected to the positive electrode of the first power supply 610-1 via the fourth switch 646.

For example, in the third time period, the controller simultaneously turns on the first switch 640 and the second switch 642, and simultaneously turns off the third switch 644 and the fourth switch 646, so that the positive electrode of the first power supply 610-1 is electrically connected with the first electrode 612-1, and the negative electrode of the first power supply 610-1 is electrically connected with the second electrode 612-2, that is, the medicament to be penetrated is introduced with the current in the first direction in the third time period. In the fourth time period, the controller simultaneously turns off the first switch 640 and the second switch 642, and simultaneously turns on the third switch 644 and the fourth switch 646, so that the negative electrode of the first power supply 610-1 is electrically connected with the first electrode 612-1, and the positive electrode of the first power supply 610-1 is electrically connected with the second electrode 612-2, that is, the medicament to be penetrated is introduced with the current in the second direction in the fourth time period. By adopting the above solution, direct current power supplies combining with multiple switches are used to solve the problem of the accumulation of capacitive charges in the skin layer, thereby improving the penetration efficiency and total amount of the medicament.

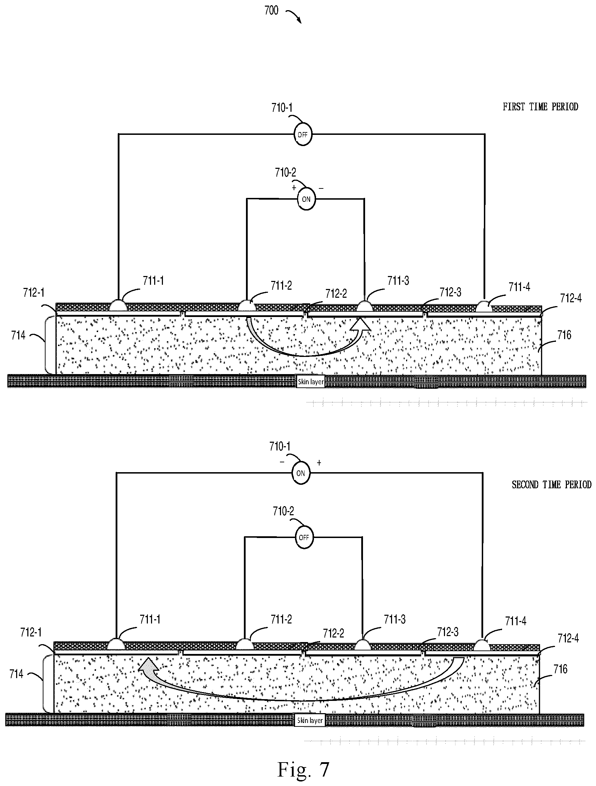

Fig. 7 shows a schematic diagram of an iontophoresis administration device 700 according to some embodiments of the present disclosure. In the iontophoresis administration device 700 of this embodiment, comprises at least a first power supply 710-1, a second power supply 710-2, a plurality of electrodes 712 (e.g., a first electrode 712-1, a second electrode 712-2, a third electrode 712-3, a fourth electrode 712-4), a plurality of connectors (e.g., comprises a first connector 711-1, a second connector 711-2, a third connector 711-3, a fourth connector 711-4), an integrated dielectric layer 714, and a controller (not shown). The integrated dielectric layer 714 has the same structure as the integrated dielectric layers shown in Figs. 1-6 and will not be described here. The first power supply 710-1 and the second power supply 710-2 are both direct current power supplies. In some embodiments, the power supplies comprised in the iontophoresis administration device 700 are all direct current power supplies.

As shown in Fig. 7, the first electrode 712-1 is electrically connected to at least one end of the first power supply 710-1 via the first connector 711-1, and the other end of the first power supply 710-1 is connected to the fourth electrode 712-1 via the fourth connector 711-4. The second electrode 712-2 is electrically connected to at least one end of the second power supply 710-2 via a second connection 711-2, and the other end of the second power supply 710-2 is connected to the third electrode 712-3 via a third connection 711-3.

For example, in a first time period, through the control of the controller (not shown), the first power supply 710-1 is turned off (that is, it works in the "OFF" state and does not output current). At the same time, the second power supply 710-2 is turned on (that is, it works in the "ON" state), the output current of the second power supply 710-2 at least partially flows through the second connector 711-2, the second electrode 712-2, at least part of the integrated dielectric layer 714, the third electrode 712-3, and the third connector 711-2 in sequence. (At this time, the positive electrode of the second power supply 710-2 is connected to the second connector 711-2. If the second power supply 710-2 is reversely connected, the current flow sequence will be different). It can be seen that in the first time period, the direction in which the direct current flows in the integrated dielectric layer 714 is as shown by the arrow in the upper half of Fig. 7, that is, the direct current in the first direction is used to introduce the medicament to be penetrated in the first time period.

For example, in a second time period, through the control of the controller (not shown), the second power supply 710-2 is turned off (that is, it works in the "OFF" state and does not output current) At the same time, the first power supply 710-1 is turned on (that is, it works in the "ON" state), the output current of the first power supply 710-1 at least partially flows through the fourth connector 711-4, the fourth electrode 712-4, at least part of the integrated dielectric layer 714, the first electrode 712-1, and the first connector 711-2 in sequence. It can be seen that in the second time period, the direction in which the direct current flows in the integrated dielectric layer 714 is as shown by the arrow in the lower half of Fig. 7, that is, the direct current in the second direction is used to introduce the medicament to be penetrated in the second time period.
By adopting the above solution, only direct current power supplies are used to solve the problem of the accumulation of capacitive charges in the skin layer, thereby improving the penetration efficiency and total amount of the medicament.

## Claims

1. An iontophoresis administration device (100, 200, 300, 400, 500, 600, 700), comprising:
a power supply (110, 310, 410, 510; 610, 710) for generating electricity required to penetrate a medicament to be penetrated (130, 230) into an administered area (122, 322) of an organism;
an integrated dielectric layer (114, 314, 414, 514, 614, 714) for covering the administered area (122, 322, 422), wherein the integrated dielectric layer comprises: a gel (118), and the polar, free-state medicament to be penetrated, wherein the gel comprises a matrix, an active medicament and an additive, the active medicament is the polar, free-state medicament to be penetrated; and
a plurality of electrodes (112, 212, 312, 412, 512, 612, 712) for electrically connecting with the power supply and the integrated dielectric layer respectively, such that the electricity generated by the power supply flows through the administered area (122) and at least part of the integrated dielectric layer respectively, wherein the at least part of the integrated dielectric layer has a predetermined resistance value (R1) the predetermined resistance value (R1) of the integrated dielectric layer is 10%~90% of skin resistance (R2) of the administered area ;
wherein the plurality of electrodes are flexible conductive electrode films separated from each other;
the iontophoresis administration device further comprises:
a back substrate layer (116, 216) for covering the plurality of electrodes, and the back substrate layer is an insulation material; and
a plurality of connectors (111, 211, 311, 411, 511, 711) for electrically connecting the power supply and the plurality of electrodes respectively, each of the plurality of connectors is at least partially disposed in the back substrate layer.

2. The device according to Claim 1, wherein the gel comprises at least one of the following components: polyethylene glycol, polyvinyl alcohol, polyhydroxyethyl methacrylate, polyacrylic acid, polymethacrylic acid, gelatin, alginic acid.

3. The device according to Claim 1, wherein the plurality of electrodes are a plurality of flexible conductive electrode films separated by a predetermined interval.

4. The device according to Claim 1, wherein the medicament to be penetrated comprises vitamin C and arbutin.

5. The device according to Claim 1, wherein the medicament to be penetrated comprises vitamin C and tranexamic acid.

6. The device according to Claim 1, wherein the medicament to be penetrated has a molecular weight less than or equal to 10,000 daltons.

7. The device according to Claim 1, wherein the thickness of the integrated dielectric layer is less than or equal to 50 mm.

8. The device according to Claim 1, wherein the electricity is an alternating current and the current intensity of the electricity is less than or equal to 5 mA.

9. The device according to Claim 1, wherein the electricity is a direct current and the current intensity of the electricity is less than or equal to 5 mA.

10. The device according to any one of Claims 8 and 9, wherein the current intensity of the electricity is greater than or equal to 0.01 mA.

11. The device according to Claim 1, wherein the power supply comprises at least a first power supply (110, 310-1, 410-1, 510-1, 610-1, 710-1), and the plurality of electrodes comprises at least a first electrode (112-1, 212-1, 312-1, 412-1, 512-1, 612-1, 712-1) and a second electrode (112-2, 212-2, 312-2, 412-2, 512-2, 612-2, 712-2), wherein the first electrode is electrically connected with a first end of the first power supply and the second electrode is electrically connected with a second end of the first power supply.

12. The device according to Claim 11, wherein the power supply further comprises a second power supply (310-2, 410-2, 510-2, 610-2, 710-2), and the plurality of electrodes further comprises a third electrode (312-3, 712-3) and a fourth electrode (312-4, 712-4), wherein the third electrode is electrically connected with a first end of the second power supply and the fourth electrode is electrically connected with a second end of the second power supply.

13. The device according to Claim 11, wherein the power supply further comprises a third power supply (410-3) and a fourth power supply (410-4), and the plurality of electrodes further comprises a fifth electrode (412-5), wherein a first end of the third power supply is electrically connected with the second end of the first power supply, a second end of the third power supply is electrically connected with the fifth electrode, a first end of the fourth power supply is electrically connected with the first end of the first power supply, and a second end of the fourth power supply is electrically connected with the second end of the third power supply.

14. The device according to Claim 11, wherein the iontophoresis administration device comprises a plurality of power supplies, the plurality of power supplies are connected in series and/or in parallel with each other for providing appropriate voltage and current to penetrate the medicament to be penetrated into the administered area .

15. The device according to Claim 1, wherein the predetermined resistance value is greater than 100 ohms and less than or equal to 100K ohms.

16. The device according to claim 15, wherein the predetermined resistance value is greater than 1K ohms and less than or equal to 12K ohms.

17. The device according to Claim 1, wherein the gel comprises collagen.

## Patentansprüche

1. Eine Iontophorese-Verabreichungsvorrichtung (100, 200, 300, 400, 500, 600, 700), die Folgendes umfasst:
eine Stromversorgung (110, 310, 410, 510; 610, 710) zur Erzeugung von Elektrizität, die erforderlich ist, um ein einzudringendes Medikament (130, 230) in einen Verabreichungsbereich (122, 322) eines Organismus einzudringen;
eine integrierte dielektrische Schicht (114, 314, 414, 514, 614, 714) zum Bedecken des Verabreichungsbereichs (122, 322, 422), wobei die integrierte dielektrische Schicht umfasst: ein Gel (118) und das polare Medikament im freien Zustand, das eindringen soll, wobei das Gel eine Matrix, ein aktives Medikament und einen Zusatzstoff umfasst, wobei das aktive Medikament das polare Medikament im freien Zustand ist, das eindringen soll; und
eine Vielzahl von Elektroden (112, 212, 312, 412, 512, 612, 712) zum elektrischen Verbinden mit der Stromversorgung und der integrierten dielektrischen Schicht jeweils, so dass die von der Stromversorgung erzeugte Elektrizität durch den Verabreichungsbereich (122) und mindestens durch einen Teil der integrierten dielektrischen Schicht jeweils fließt, wobei der mindestens eine Teil der integrierten dielektrischen Schicht einen vorbestimmten Widerstandswert (R1) aufweist, wobei der vorbestimmte Widerstandswert (R1) der integrierten dielektrischen Schicht 10%~90% des Hautwiderstands (R2) des Verabreichungsbereichs beträgt;
wobei die Vielzahl von Elektroden flexible, leitfähige und voneinander getrennte Elektrodenfilme sind;
die Iontophorese-Verabreichungsvorrichtung umfasst ferner:
eine hintere Substratschicht (116, 216) zum Bedecken der Vielzahl von Elektroden, wobei die hintere Substratschicht ein Isoliermaterial ist; und
eine Mehrzahl von Verbindern (111, 211, 311, 411, 511, 711) zum elektrischen Verbinden der Stromversorgung und der Mehrzahl von Elektroden jeweils, wobei jeder der Vielzahl von Verbindern zumindest teilweise in der hinteren Substratschicht angeordnet ist.

2. Vorrichtung nach Anspruch 1, wobei das Gel mindestens eine der folgenden Komponenten umfasst: Polyethylenglykol, Polyvinylalkohol, Polyhydroxyethylmethacrylat, Polyacrylsäure, Polymethacrylsäure, Gelatine, Alginsäure.

3. Vorrichtung nach Anspruch 1, wobei die Vielzahl von Elektroden eine Vielzahl von flexiblen, leitfähigen Elektrodenfilmen ist, die durch einen vorbestimmten Abstand getrennt sind.

4. Vorrichtung nach Anspruch 1, wobei das einzudringende Medikament Vitamin C und Arbutin umfasst.

5. Vorrichtung nach Anspruch 1, wobei das einzudringende Medikament Vitamin C und Tranexamsäure umfasst.

6. Vorrichtung nach Anspruch 1, wobei das einzudringende Medikament ein Molekulargewicht von 10.000 Dalton oder weniger aufweist.

7. Vorrichtung nach Anspruch 1, wobei die Dicke der integrierten dielektrischen Schicht kleiner oder gleich 50 mm ist.

8. Vorrichtung nach Anspruch 1, wobei der Strom ein Wechselstrom ist und die Stromstärke des Stroms kleiner oder gleich 5 mA ist.

9. Vorrichtung nach Anspruch 1, wobei der Strom ein Gleichstrom ist und die Stromstärke des Stroms kleiner oder gleich 5 mA ist.

10. Vorrichtung nach einem der Ansprüche 8 und 9, wobei die Stromstärke des Stroms größer oder gleich 0,01 mA ist.

11. Vorrichtung nach Anspruch 1, wobei die Stromversorgung mindestens eine erste Stromversorgung (110, 310-1, 410-1, 510-1, 610-1, 710-1) umfasst, und die Vielzahl von Elektroden mindestens eine erste Elektrode (112-1, 212-1, 312-1, 412-1, 512-1, 612-1, 712-1) und eine zweite Elektrode (112-2, 212-2, 312-2, 412-2, 512-2, 612-2, 712-2) umfasst, wobei die erste Elektrode elektrisch mit einem ersten Ende der ersten Stromversorgung und die zweite Elektrode elektrisch mit einem zweiten Ende der ersten Stromversorgung verbunden ist.

12. Vorrichtung nach Anspruch 11, wobei die Stromversorgung ferner eine zweite Stromversorgung (310-2, 410-2, 510-2, 610-2, 710-2) umfasst, und die Vielzahl von Elektroden ferner eine dritte Elektrode (312-3, 712-3) und eine vierte Elektrode (312-4, 712-4) umfasst, wobei die dritte Elektrode elektrisch mit einem ersten Ende der zweiten Stromversorgung verbunden ist und die vierte Elektrode elektrisch mit einem zweiten Ende der zweiten Stromversorgung verbunden ist.

13. Vorrichtung nach Anspruch 11, wobei die Stromversorgung ferner eine dritte Stromversorgung (410-3) und eine vierte Stromversorgung (410-4) umfasst, und die Mehrzahl von Elektroden ferner eine fünfte Elektrode (412-5) umfasst, wobei ein erstes Ende der dritten Stromversorgung elektrisch mit dem zweiten Ende der ersten Stromversorgung verbunden ist, ein zweites Ende der dritten Stromversorgung elektrisch mit der fünften Elektrode verbunden ist, ein erstes Ende der vierten Stromversorgung elektrisch mit dem ersten Ende der ersten Stromversorgung verbunden ist und ein zweites Ende der vierten Stromversorgung elektrisch mit dem zweiten Ende der dritten Stromversorgung verbunden ist.

14. Vorrichtung nach Anspruch 11, wobei die Iontophorese-Verabreichungsvorrichtung eine Vielzahl von Stromversorgungen umfasst, wobei die Vielzahl von Stromversorgungen in Reihe und/oder parallel zueinander geschaltet sind, um eine geeignete Spannung und einen geeigneten Strom bereitzustellen, um das zu einzudringende Medikament in den Verabreichungsbereich einzudringen.

15. Vorrichtung nach Anspruch 1, wobei der vorbestimmte Widerstandswert größer als 100 Ohm und kleiner als oder gleich 100K Ohm ist.

16. Vorrichtung nach Anspruch 15, wobei der vorbestimmte Widerstandswert größer als 1K Ohm und kleiner als oder gleich 12K Ohm ist.

17. Verfahren nach Anspruch 1, wobei das Gel Kollagen umfasst.

## Revendications

1. Dispositif d'administration par iontophorèse (100, 200, 300, 400, 500, 600, 700), comprenant :
une alimentation électrique (110, 310, 410, 510 ; 610, 710) pour générer l'électricité nécessaire pour faire pénétrer un médicament à faire pénétrer (130, 230) dans une zone d'administration (122, 322) d'un organisme ;
une couche diélectrique intégrée (114, 314, 414, 514, 614, 714) pour recouvrir la zone d'administration (122, 322, 422), dans lequel la couche diélectrique intégrée comprend : un gel (118), et le médicament polaire à l'état libre à faire pénétrer, dans lequel le gel comprend une matrice, un médicament actif et un additif, le médicament actif étant le médicament polaire à l'état libre à faire pénétrer ; et
une pluralité d'électrodes (112, 212, 312, 412, 512, 612, 712) destinées à être respectivement connectées électriquement à l'alimentation électrique et à la couche diélectrique intégrée, de sorte que l'électricité générée par l'alimentation électrique circule à travers la zone d'administration (122) et au moins une partie de la couche diélectrique intégrée respectivement, dans lequel l'au moins une partie de la couche diélectrique intégrée a une valeur de résistance prédéterminée (R1), la valeur de résistance prédéterminée (R1) de la couche diélectrique intégrée est de 10 %~90 % de la résistance de la peau (R2) de la zone d'administration ;
dans lequel la pluralité d'électrodes sont des films d'électrode conducteurs souples séparés les uns des autres ;
le dispositif d'administration par iontophorèse comprend en outre :
une couche de substrat arrière (116, 216) pour recouvrir la pluralité d'électrodes, et la couche de substrat arrière est un matériau isolant ; et
une pluralité de connecteurs (111, 211, 311, 411, 511, 711) pour connecter électriquement l'alimentation électrique et la pluralité d'électrodes respectivement,
chacun de la pluralité de connecteurs est au moins partiellement disposé dans la couche de substrat arrière.

2. Dispositif selon la revendication 1, dans lequel le gel comprend au moins un des composants suivants : polyéthylène glycol, alcool polyvinylique, méthacrylate de polyhydroxyéthyle, acide polyacrylique, acide polyméthacrylique, gélatine, acide alginique.

3. Dispositif selon la revendication 1, dans lequel la pluralité d'électrodes est constituée d'une pluralité de films d'électrode conducteurs souples séparés par un intervalle prédéterminé.

4. Dispositif selon la revendication 1, dans lequel le médicament à faire pénétrer comprend de la vitamine C et de l'arbutine.

5. Dispositif selon la revendication 1, dans lequel le médicament à faire pénétrer comprend de la vitamine C et de l'acide tranexamique.

6. Dispositif selon la revendication 1, dans lequel le médicament à faire pénétrer a un poids moléculaire inférieur ou égal à 10 000 daltons.

7. Dispositif selon la revendication 1, dans lequel l'épaisseur de la couche diélectrique intégrée est inférieure ou égale à 50 mm.

8. Dispositif selon la revendication 1, dans lequel l'électricité est un courant alternatif et l'intensité du courant électrique est inférieure ou égale à 5 mA.

9. Dispositif selon la revendication 1, dans lequel l'électricité est un courant continu et l'intensité du courant électrique est inférieure ou égale à 5 mA.

10. Dispositif selon l'une quelconque des revendications 8 et 9, dans lequel l'intensité du courant électrique est supérieure ou égale à 0,01 mA.

11. Dispositif selon la revendication 1, dans lequel l'alimentation électrique comprend au moins une première alimentation électrique (110, 310-1, 410-1, 510-1, 610-1, 710-1), et la pluralité d'électrodes comprend au moins une première électrode (112-1, 212-1, 312-1, 412-1, 512-1, 612-1, 712-1) et une deuxième électrode (112-2, 212-2, 312-2, 412-2, 512-2, 612-2, 712-2), dans lequel la première électrode est connectée électriquement à une première extrémité de la première alimentation électrique et la deuxième électrode étant connectée électriquement à une seconde extrémité de la première alimentation électrique.

12. Dispositif selon la revendication 11, dans lequel l'alimentation électrique comprend en outre une deuxième alimentation électrique (310-2, 410-2, 510-2, 610-2, 710-2), et la pluralité d'électrodes comprend en outre une troisième électrode (312-3, 712-3) et une quatrième électrode (312-4, 712-4), dans lequel la troisième électrode est connectée électriquement à une première extrémité de la deuxième alimentation électrique et la quatrième électrode est connectée électriquement à une seconde extrémité de la deuxième alimentation électrique.

13. Dispositif selon la revendication 11, dans lequel l'alimentation électrique comprend en outre une troisième alimentation électrique (410-3) et une quatrième alimentation électrique (410-4), et la pluralité d'électrodes comprend en outre une cinquième électrode (412-5), dans lequel une première extrémité de la troisième alimentation électrique est connectée électriquement à la seconde extrémité de la première alimentation électrique, une seconde extrémité de la troisième alimentation électrique est connectée électriquement à la cinquième électrode, une première extrémité de la quatrième alimentation électrique est connectée électriquement à la première extrémité de la première alimentation électrique, et une seconde extrémité de la quatrième alimentation électrique est connectée électriquement à la seconde extrémité de la troisième alimentation électrique.

14. Dispositif selon la revendication 11, dans lequel le dispositif d'administration par iontophorèse comprend une pluralité d'alimentations électriques, la pluralité d'alimentations électriques sont connectées en série et/ou en parallèle les unes avec les autres pour fournir la tension et le courant appropriés pour faire pénétrer le médicament à faire pénétrer dans la zone d'administration.

15. Dispositif selon la revendication 1, dans lequel la valeur de résistance prédéterminée est supérieure à 100 ohms et inférieure ou égale à 100K ohms.

16. Dispositif selon la revendication 15, dans lequel la valeur de résistance prédéterminée est supérieure à 1K ohms et inférieure ou égale à 12K ohms.

17. Dispositif selon la revendication 1, dans lequel le gel comprend du collagène.
